# EUROPEAN PATENT APPLICATION

(11) **EP 4 349 305 A1**
(43) Date of publication of application: **10.04.2024**
(21) Application number: 23185897.8
(22) Date of filing: 17.07.2023
(51) Int. Cl.: A61F 2/30, A61F 2/32, A61F 2/38, A61F 2/40, A61F 2/42, A61F 2/46

(54) **PROSTHESES COUPLING SYSTEMS**

(30) Priority: 07.10.2022 US 202263378714 P; 07.07.2023 US 202318348513
(71) Applicant: Wright Medical Technology, Inc., Memphis, TN 38117 (US)
(72) Inventor: PENNER, Murray J., Vancouver, V6B6C1 (CA); LUNA, Ramon, Arlington, 38002 (US); McGEE, Johnny, Halls, 38040 (US); MOORE, Jesse G., Germantown, 38138 (US)
(74) Representative: Zimmermann & Partner Patentanwälte mbB

(57) **Abstract**

Various systems are disclosed for joining at least two components of a prosthesis. For example, a system (100) may include a first component (260) having a first a first body (202a) extending from a first face (208a) to a second face (210a). An engagement element (216a) may be disposed on the first face of the first body and may be configured to engage a second component (270). A biasing member (238a) may be disposed on the engagement element and may be configured to latch a second component to the first component. A securing mechanism (232a) may be configured to receive a locking mechanism (234a) at an angle with respect to a longitudinal axis. The locking mechanism may be adapted to interact with the biasing member.

## Description

### CROSS-REFERENCE TO RELATED APPLICATION

This application claims priority under 35 U.S.C. § 119(e) to U.S. Provisional Application No. 63/378,714, filed October 7, 2022, the entirety of which is incorporated herein by reference.

### INCORPORATION BY REFERENCE

This application is related to commonly owned U.S. patent application Ser. No. 12/410,978 filed on March 25, 2009, now U.S. Patent No. 8,715,362, U.S. patent application Ser. No. 16/605,849, which is a U.S. national phase entry under 35 U.S.C. § 371 of international patent application No. PCT/US2017/040730 filed on July 5, 2017, U.S. patent application Ser. No. 17/650,116 filed on February 7, 2022 and U.S. Provisional Application No. 63/269,269 filed on March 14, 2022 the entireties of which are incorporated by reference herein.

### FIELD OF DISCLOSURE

The disclosed systems and methods relate to implants. More particularly, the disclosed systems and methods relate to the coupling and decoupling of two or more stemmed components of a prosthesis or implant.

### BACKGROUND

Medical prostheses are available to address any number of abnormalities. For example, a prosthesis may be provided to replace a joint, such as a shoulder, elbow, knee, or ankle. Each prosthesis may include one or more components, such as a stem that is to be inserted into a passageway formed along an axis of a bone, and a tray that is to be coupled to the stem. A tray may support one or more additional components, such as an articular surface formed from metal or polymer that may be coupled to the tray. In order to speed recovery and reduce complications, surgical techniques seek to minimize the size or length of an incision or access site needed to install a prosthesis and maximize the efficiency of the operation. As a result, there continues to be a need to minimize the size of the implant to be installed and improve the efficiency of the operation.

### SUMMARY

In some embodiments, a system may include a first component, an engagement element, a biasing member, and a securing mechanism. The first component may have a first body extending from a first face to a second face. The engagement element may be disposed on the first face of the first body and may be configured to engage a second component. The biasing member may be disposed on the engagement element and may be configured to latch a second component to the first component. The securing mechanism may be configured to receive a locking mechanism at an angle with respect to a longitudinal axis. The locking mechanism may be adapted to interact with the biasing member.

In some embodiments, a method may include coupling a first component to a second component by aligning a pair of rails on the first component to a pair of rails on the second component sliding the second component in a posterior direction to engage the pair of rails of the second component with the pair of rails on the first component.

In some embodiments, a method may include decoupling a first component from a second component. The first and second components may both include a respective body. The respective bodies may include respective spaced apart rails of the first and second component. The decoupling may include unlocking the first and second components by uninstalling a locking mechanism from a securing mechanism disposed, at an angle with respect to a longitudinal axis of the first component on an outer surface of the first component. The removal of the locking mechanism may move a biasing member into a second position. The decoupling may include moving the first and second components apart by sliding the respective spaced apart rails away from each other by pulling an extraction tool handle in a posterior direction.

In some embodiments, a system may include a first component, a pair of rails, and a biasing member. The first component may include a first body extending from a first face to a second face. The pair of rails may be disposed on the first face of the first body. The pair of rails may be configured to engage a second component. The biasing member may be disposed on the first face of the first body and may be configured to latch the first and second component together.

In some embodiments, a method may include coupling a first component and a second component together by aligning a respective pair of rails of the first and second components and locking the first and second components together with an engagement element disposed on a first face of the second component. The engagement element may include a tab, a cut out, an angled slope, and a ledge. The tab may be configured to fit inside of a detent. The cut out on the bottom side of the tab may be configured to allow the tab to bend. The angled slope may be adapted to allow a removal tool access. The ledge may be on the first end of the tab. The ledge may be adapted to lock inside of a detent on a bottom end of the first component.

In some embodiments, a method may include decoupling a first component from a second component. The first and second components may include a respective body. The respective bodies may include respective spaced apart rails. The decoupling may include sliding a removal tool along an angled slope on the body of the second component. The removal tool may be adapted to engage a tab on the second component and may remove the tab from a detent on a second face of the first component. If the tab is removed from the detent, moving the respective spaced apart rails of the first and second component apart by pulling an extraction tool handle in a posterior direction completing the decoupling.

Further disclosed herein are systems and methods for joining at least two components of a prosthesis, wherein a system includes a first component having a first a first body extending from a first face to a second face, wherein an engagement element is disposed on the first face of the first body and is configured to engage a second component, wherein a biasing member is disposed on the engagement element and is configured to latch a second component to the first component, wherein a securing mechanism is configured to receive a locking mechanism at an angle with respect to a longitudinal axis, and wherein the locking mechanism may be adapted to interact with the biasing member.

### BRIEF DESCRIPTION OF THE DRAWINGS

FIG. 1 is an isometric view of an example of a prosthesis system having a coupling mechanism in accordance with some embodiments.
FIG. 2 is an isometric view of the stem components of the system illustrated in FIG. 1 in accordance with some embodiments;
FIG. 3 is an isometric view of stem components of the system illustrated in FIG. 1 in accordance with some embodiments.
FIG. 4 is an isometric view of the middle stem component of the system illustrated in FIG. 1 in accordance with some embodiments.
FIG. 5 is a top side perspective view of the middle stem component of the system illustrated in FIG. 1 in accordance with some embodiments.
FIG. 6 is an isometric view of stem components of the system illustrated in FIG. 1 configured to allow removal with an extraction tool in accordance with some embodiments.
FIG. 7 is an isometric view of stem components of the system illustrated in FIG. 6 including an extraction tool in accordance with some embodiments.
FIG. 8 is a cross-sectional view of stem components illustrated in FIG. 7, in a first position, taken along line 8-8 in FIG. 7 in accordance with some embodiments.
FIG. 9 is a cross-sectional view of stem components illustrated in FIG. 7, in a second position, taken along line 8-8 in FIG. 7 in accordance with some embodiments.
FIG. 10 is an isometric view of an example of a prosthesis system having a coupling mechanism in accordance with some embodiments.
FIG. 11 is an isometric view of stem components illustrated in FIG. 10 in accordance with some embodiments.
FIG. 12 is a cross-sectional view of stem components illustrated in FIG. 11 taken along line 12-12 in FIG. 11 in accordance with some embodiments.
FIG. 13 is an isometric view of the top and the middle stem components of the system illustrated in FIG. 10 in accordance with some embodiments.
FIG. 14 is an exploded view of the stem components illustrated in FIG. 3 in accordance with some embodiments.
FIG. 15 is a cross-sectional view of the stem components illustrated in FIG. 13, in a first position, taken along line 15-15 in FIG. 13 in accordance with some embodiments.
FIG. 16 is a cross-sectional view of the stem components illustrated in FIG. 13, in a second position, taken along line 15-15 in FIG. 13 in accordance with some embodiments.
FIG. 17 is a cross-sectional view of the stem components illustrated in FIG. 13, in a third position, taken along line 15-15 in FIG. 13 in accordance with some embodiments.
FIG. 18 is a cross-sectional view of the stem components illustrated in FIG. 13, in a fourth position, taken along line 15-15 in FIG. 13 in accordance with some embodiments.
FIG. 19 is an isometric view of stem components of the system illustrated in FIG. 10 configured to allow removal with an extraction tool in accordance with some embodiments.
FIG. 20 is an isometric view of stem components of the system illustrated in FIG. 19 including an extraction tool in accordance with some embodiments.
FIG. 21 is a cross-sectional view of stem components illustrated in FIG. 20, in a first position, taken along line 21-21 in FIG. 20 in accordance with some embodiments.
FIG. 22 is a cross-sectional view of stem components illustrated in FIG. 20, in a second position, taken along line 21-21 in FIG. 20 in accordance with some embodiments.
FIG. 23 is an isometric view of an example of a prosthesis system having a coupling mechanism in accordance with some embodiments.
FIG. 24 is an isometric view of the stem components of the system illustrated in FIG. 23 in accordance with some embodiments.
FIG. 25 is an isometric view of stem components of the system illustrated in FIG. 23 in accordance with some embodiments.
FIG. 26 is an isometric view of the middle stem component of the system illustrated in FIG. 23 in accordance with some embodiments.
FIG. 27 is a cross-sectional view of the stem component illustrated in FIG. 26 taken along line 27-27 in FIG. 26 in accordance with some embodiments.
FIG. 28 is a cross-sectional view of the stem components illustrated in FIG. 25 taken along line 28-28 in FIG. 25 in accordance with some embodiments.
FIG. 29 is a side perspective view of the stem component illustrated in FIG. 26 according to some embodiments.
FIG. 30 is an isometric view of stem components of the system illustrated in FIG. 23 configured to allow removal with an extraction tool in accordance with some embodiments.
FIG. 31 is an isometric view of stem components of the system illustrated in FIG. 30 including an extraction tool in accordance with some embodiments.
FIG. 32 is a cross-sectional view of stem components illustrated in FIG. 31, in a first position, taken along line 32-32 in FIG. 31 in accordance with some embodiments.
FIG. 33 is a cross-sectional view of stem components illustrated in FIG. 31, in a second position, taken along line 32-32 in FIG. 31 in accordance with some embodiments.

### DETAILED DESCRIPTION

This description of the exemplary embodiments is intended to be read in connection with the accompanying drawings, which are to be considered part of the entire written description.

The disclosed components, systems, and methods provide for coupling together two or more components of a medical prosthesis and facilitate the coupling together of the various components. The disclosed components, systems, and methods also provide for decoupling two or more components of a medical prosthesis to facilitate removal of the components. In some embodiments, the components may be coupled together in situ through a minimally invasive opening, although it should be understood that the components may be coupled together ex situ and implanted as a single construct. In some embodiments, two or more components may be assembled ex situ to form a first assembly, and at least one third component may be assembled to the first assembly in situ to form a second assembly. One or more fourth components may be assembled to the second assembly in situ. Although the following descriptions are provided with reference to an ankle prosthesis, such as the INBONE^{™} Total Ankle System available from the Wright Medical Group and the ankle prostheses disclosed in U.S. Patent No. 8,715,362, entitled "Ankle Replacement System," which is incorporated by reference herein in its entirety, it should be understood that the disclosed systems and methods are not to be limited to such prosthesis and may be used in connection with any number of different prosthesis, including prostheses for joints other than ankles. For example, the disclosed components, systems, and methods may be applied to prosthesis for other joints including, but not limited, hips, shoulders, elbows, and knees, to list only a few possibilities.

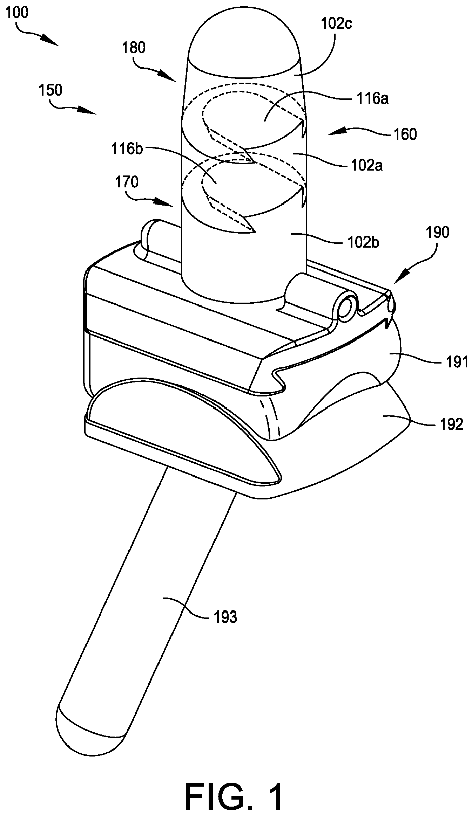

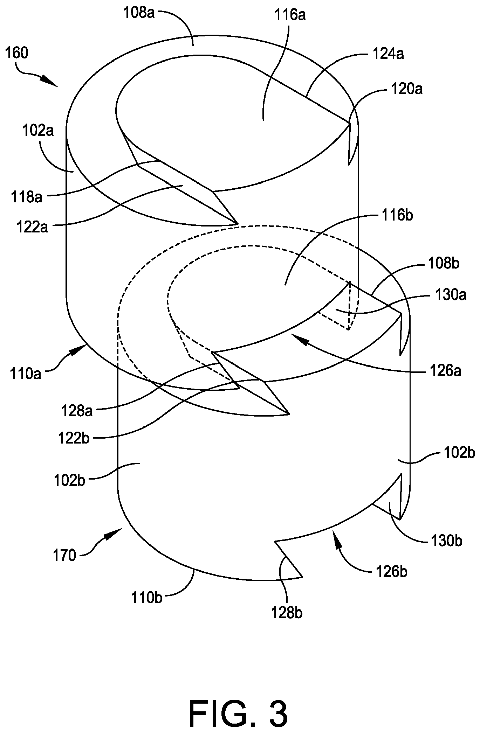

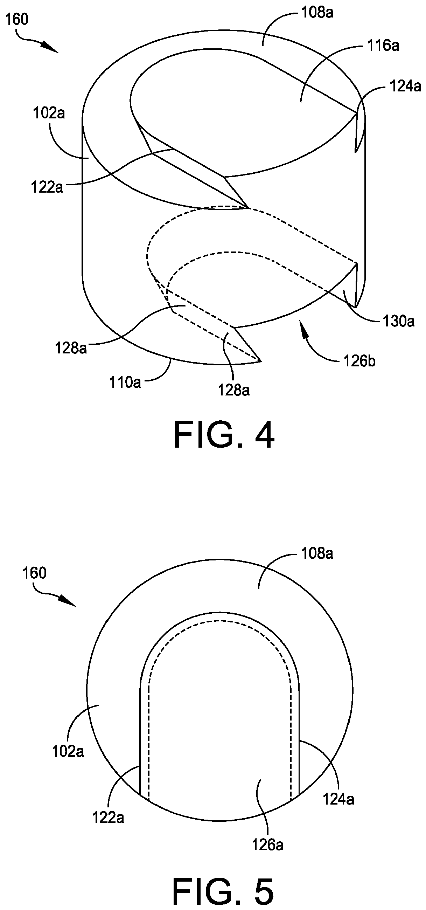

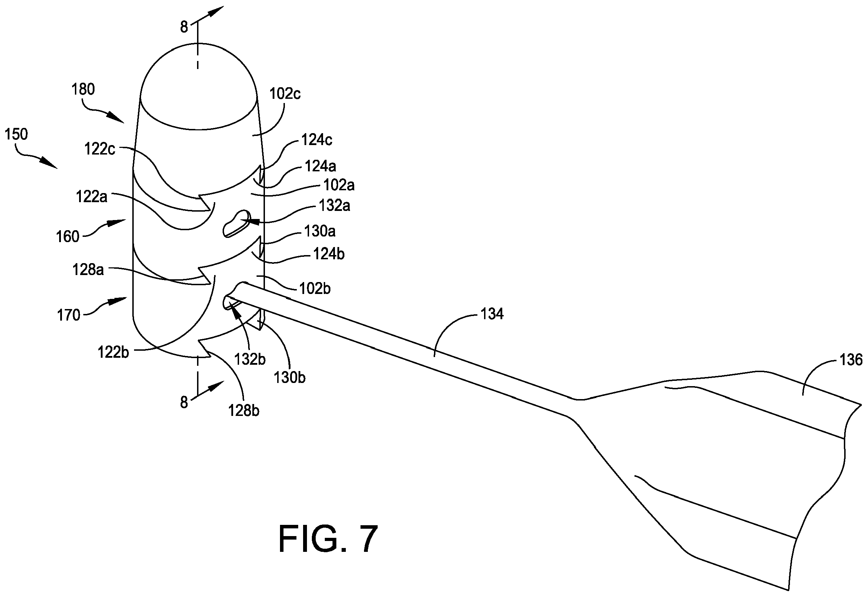

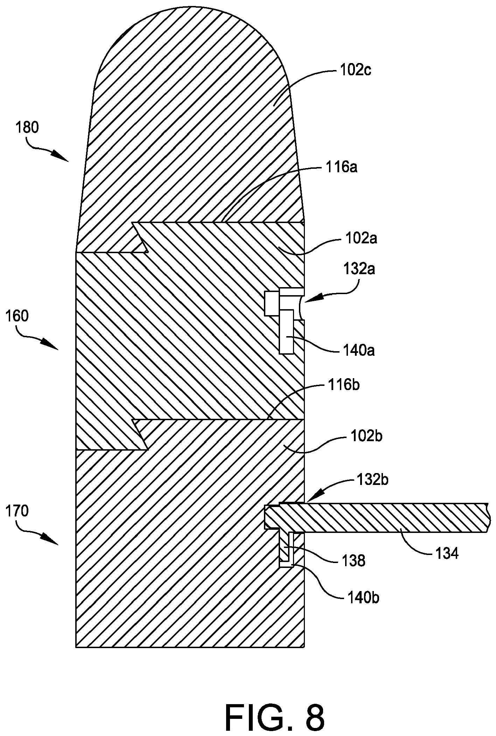

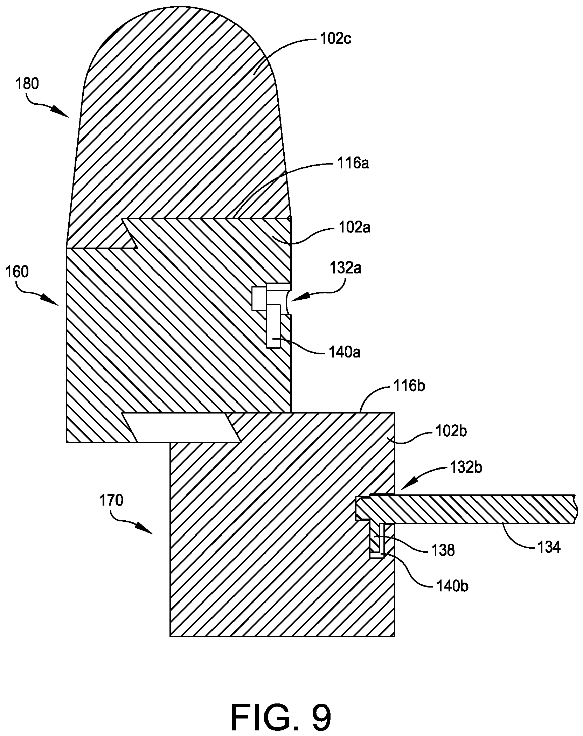

FIGS. 1-9 illustrate one example of a system 100 including a coupling mechanism in accordance with some embodiments. In some embodiments, the system 100 may be an ankle prosthesis including a stem 150, a tibia tray component 190, a tibial artificial joint surface 191, a talar component 192 with artificial surface, and a talar stem 193. The stem 150 may be a multi-component stem including two or more components that may be coupled by a coupling mechanism, which may be a dovetail coupling mechanism, as described herein. Although the components described herein are described with reference to an ankle prosthesis, the components, systems, and methods are not so limited, as one of ordinary skill in the art will understand that the components may be adapted for other prosthesis, including the hip, knee, and shoulder, to list only a few possible examples.

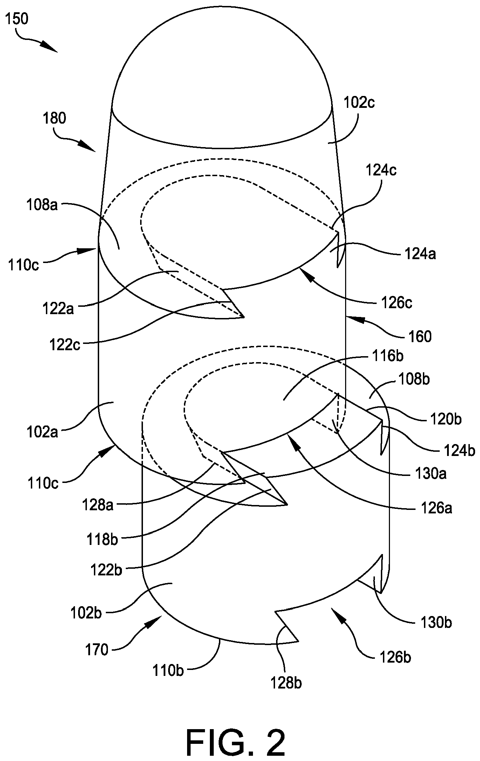

In the example illustrated in FIG. 2, the stem 150 includes a first component 180, which may be a top or leading component, a second component 160, which may be a middle component, and a third component 170, which may be a bottom component or another middle component. It should be understood that the second and third components may be identical or different depending on the length and configuration of the stem, as described herein. In some embodiments, the components 160, 170, 180 may be coupled together either in situ or ex situ.

Each of the stem components 160, 170, 180 may include a body 102a, 102b, 102c having a generally cylindrical shape, with stem component 180 tapering along its length from a first diameter at leading end to a second diameter at its trailing end. In some embodiments, the second diameter is greater than the first diameter. It should be understood that stem components 160, 170, 180 may have other shapes or configurations (e.g., conical, pyramidal, cubic, and/or oval, to list only a few examples). In addition, the stem components 160, 170, 180 could be arched to reduce and/or prevent rotation, and could be of constant or varying cross-sectional widths. In some embodiments, the stem components 160, 170, 180 may include one or more anti-rotation features extending radially outward from the body 102a, 102b, 102c, as will be understood by one of ordinary skill in the art. Examples of such anti-rotation features are described in U.S. Patent Application Publication No. 2022/0280307 and U.S. Provisional Application No. 63/374,248, filed September 1, 2022, which are incorporated by reference herein in its entirety. Body 102a, 102b, 102c may be formed using an additive manufacturing process, such as Direct Metal Laser Sintering (DMLS) or Electron Beam Melting (EBM), to list only a few possibilities. Other conventional manufacturing processes, such as machining, molding, extruding, and combinations thereof, may also be used as will be understood by one of ordinary skill in the art.

In some embodiments, body 102c of leading component 180 further include a second face 110c. The second face 110c may define a pair of spaced apart rails 122c and 124c, disposed within channel 126c, configured to slide along spaced apart rails of another prosthesis component (e.g., stem component 160, 170 and/or another stem component of a multi-component stem) to form a dovetail coupling.

In some embodiments, the body 102a of stem component 160 may further include a first face 108a and a second face 1 10a. The first face 108a may, in some embodiments, define an engagement element 116a, such as a shelf or one or more flats, having a first edge 118a and a second edge 120a. The first 118a and second 120a edges includes spaced apart rails, 122a and 124a respectively, configured to slide along spaced apart rails within a channel disposed on the second face of another prosthesis component (e.g., prosthesis component 160, 170 and/or another stem component, such as leading component 180, of a multi-component stem) to form a dovetail coupling. The second face 110a, in some embodiments, further includes a pair of spaced apart rails 128a and 130a, disposed within channel 126a, configured to slide along spaced apart rails of another prosthesis component (e.g., stem component 160, 170 and/or another stem component of a multi-component stem) to form a dovetail coupling. One of ordinary skill in the art will understand that other engagement elements may be used instead of flats, rails, or shelfs.

In some embodiments, the body 102b of stem component 170 may further include a first face 108b and a second face 110b. The first face 108b may, in some embodiments, define an engagement element 116b, such as a shelf or one or more flats, having a first edge 118b and a second edge 120b. The first 118b and second 120b edges includes spaced apart rails, 122b and 124b respectively, configured to slide along spaced apart rails within a channel disposed on the second face of another prosthesis component (e.g., prosthesis component 160, 170 and/or another stem component, such as leading component 180, of a multi-component stem) to form a dovetail coupling. The second face 110b, in some embodiments, further includes a pair of spaced apart rails 128b and 130b, disposed within channel 126b, configured to slide along spaced apart rails of another prosthesis component (e.g., stem component 160, 170 and/or another stem component of a multi-component stem) to form a dovetail coupling. One of ordinary skill in the art will understand that other engagement elements may be used instead of flats, rails, or shelfs.

In some embodiments, a leading component 180, may be attached to components 160 and/or 170. For example, the body 102c of component 180 may be inserted into a resected joint space between first and second bones, which may be a tibia and a talus. The component 180 may be advanced, at least partially, into an intramedullary canal form in one of the bones (e.g., the tibia). Component 160 may then be coupled to the component 180 by aligning spaced apart rails 122a, 124a and 122c, and 124c of the two components 160, 180. Once aligned, component 160 may be pushed into engagement (e.g., by pushing in a first direction such as backward or in a posterior direction) so that engagement element 116a on the first face 108a of component 160 contacts the back end of the channel 126c. In some embodiments, a locking mechanism, such as a set screw, may be placed in an opening penetrating a side of the body 102c and into engagement element 116a locking the two components 160, 180 together. Stem component 160 may be installed by hand and/or by using a tool fitted around the body to install the two components 160, 180 together. One of ordinary skill in the art will understand that other engagement elements may be used, such as a spring-loaded detent or a depression and projections.

Bottom or middle component 170 may be coupled to middle component 160 by aligning the spaced apart rails 128a, 130a and 122b, 124b of the two components 160,170. Once aligned, component 170 may be pushed into engagement (e.g., by pushing in a first direction such as backward or in a posterior direction) so that engagement element 116b on the first face of component 170 contacts the back end of the channel 126a. In some embodiments, a locking mechanism, such as a set screw, may be placed in an opening penetrating a side of the body 102a and into the engagement element 116b locking the two components 160, 170 together. Stem component 160 may be installed by hand and/or by using a tool fitted around the body to install the two components 160, 170 together. One of ordinary skill in the art will understand that other engagement elements may be used, such as a spring-loaded detent or a depression and projections.

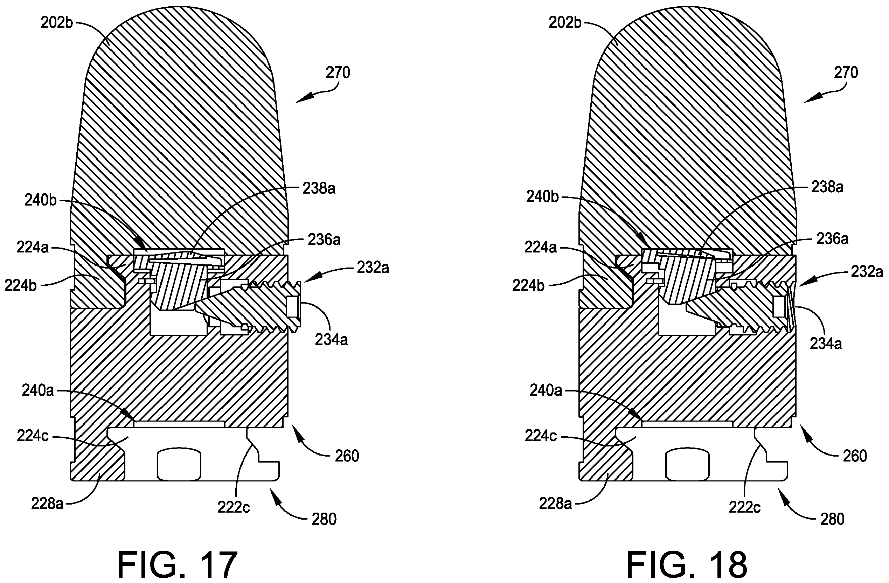

In some embodiments, another component may be coupled to components 160 and/or 170, such as a tibial tray component 190 for an ankle replacement system similar to the tibial platform 12 described in reference to FIG. 17 of U.S. Patent No. 8,715,362. Other components, such as one or of the tibia tray components described in U.S. Patent Application No. 17/650,116, may also be used. In some embodiments, the tibial tray component 190 may define a channel for receiving an artificial articular surface 191, which may be configured to engage another articular surface, such as a talus or another artificial articular surface 192, as shown in FIG. 1. In some embodiments, one of the stem components 160, 170, and/or 180 may be coupled to the tray component 190 by aligning spaced apart rails of the stem component 160, 170, and/or 180 with an engagement element disposed on the tray component 190 similar to the coupling described above for the coupling of components 160 and 170, 180. In other embodiments, tray component 190 may be coupled to another stem component 160, 170, and/or 180 with a tapered connection. In the embodiment with a tapered connection for the tray component 190, a modified stem component, such as component 160, 170, or 180, would be provided without the channel and spaced apart rails as described above and would be adapted to fit with the tapered connection of the tray component 190.

Component 170 may be decoupled from component 160 by removing the locking mechanism (e.g., removing the set screw if applicable) and pulling the component 170 forward (e.g., in an anterior direction) along the spaced apart rails 128a, 130a and 122b, 124b of both components 160, 170. In some embodiments, a removal tool (not shown) may be used to remove the locking mechanism or force the component 170 out of contact with the back end of the channel 126a as will be understood by one of ordinary skill in the art.

Component 180 may be decoupled from component 160 by removing the locking mechanism (e.g., removing the set screw if applicable) and pulling the component 160 forward (e.g., in an anterior direction) along the spaced apart rails 122a, 124a and 122c, 124c of both components 160, 180. In some embodiments, a removal tool (not shown) may be used to remove the locking mechanism or force the component 160 out of contact with the back end of the channel 126c as will be understood by one of ordinary skill in the art.

In some embodiments, the decoupling and removal of the tibial tray component 190, from at least one other component (e.g., component 160, 170, and/ or 180) allows access to the internal engagement features of at least one other component 160, 170, 180. Removal of the tibial tray component 190, may be achieved with an impacting instrument(s) in a distal or caudal direction as will be understood by one of ordinary skill in the art. The internal engagement features of the components 160, 170, 180 may be spaced apart rails, such as the spaced apart rails 128a, 130a of component 160, and may be configured to engage a removal instrument (not shown). The removal instrument may engage the internal engagement features of components 160, 170, and/or 180, such as the spaced apart rails 128a, 130a of component 160, to facilitate removal. It is understood that the internal engagement features may also include holes, tabs, threads, and/or recesses to facilitate engagement with the removal instrument(s).

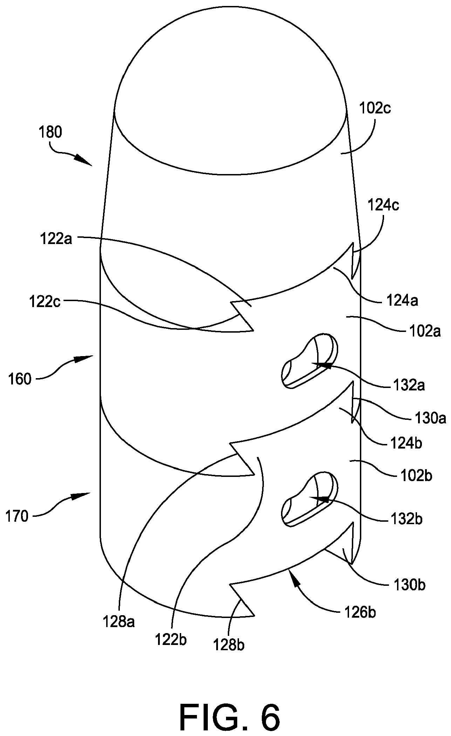

As best seen in FIGS. 6-9, stem components may include an extraction opening 132a, 132b in the body 102a, 102b configured to fit a separate tool, such as extraction tool 134, to allow for the decoupling and removal of at least one component, such as components 160, 170, and/or 180. In some embodiments, the extraction tool 134 may include a handle 136 disposed on a first end of the extraction tool 134 and an extraction mechanism 138 disposed on a second end of the extraction tool 134. The extraction mechanism 138, may in some embodiments, be a protrusion that extends at an angle from the shaft and adapted to fit inside of a recess 140a, 140b disposed inside of the extraction opening 132a 132b on body 102a, 102b. In some embodiments, the decoupling and removal of the stem components may include inserting the extraction tool 134 and extraction mechanism 138 into the extraction opening 132a, 132b and the recess 140a, 140b disposed on body 102a, 102b. Removal may then be facilitated by pulling away from (or in the posterior direction) the stem allowing the stem components to move along the spaced apart rails, such as spaced apart rails 128a, 130a and 122b, 124b of components 160 and 170 respectively.

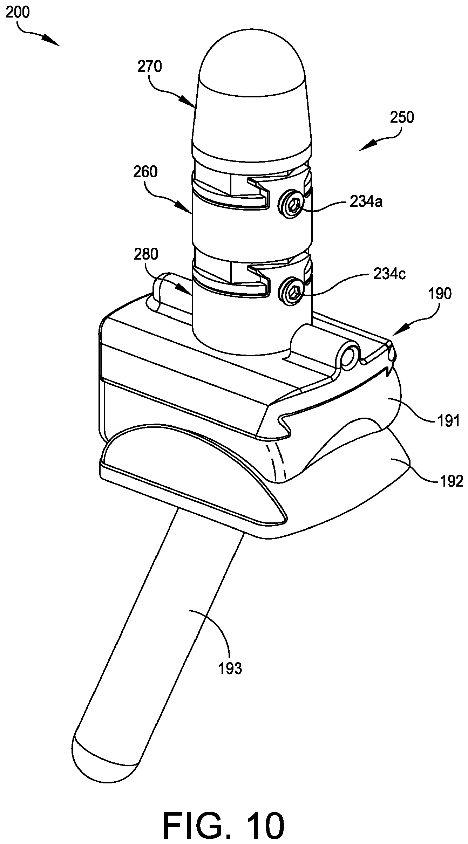

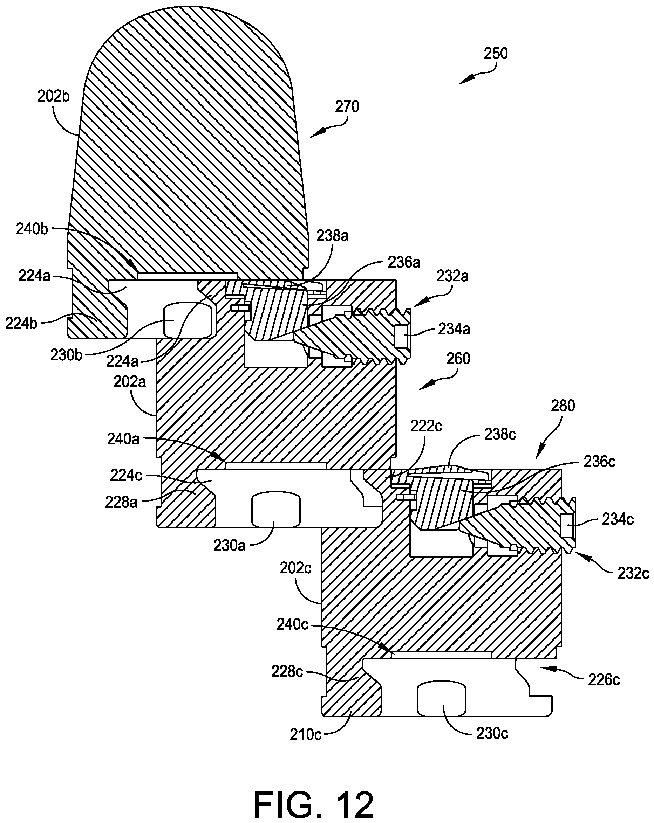

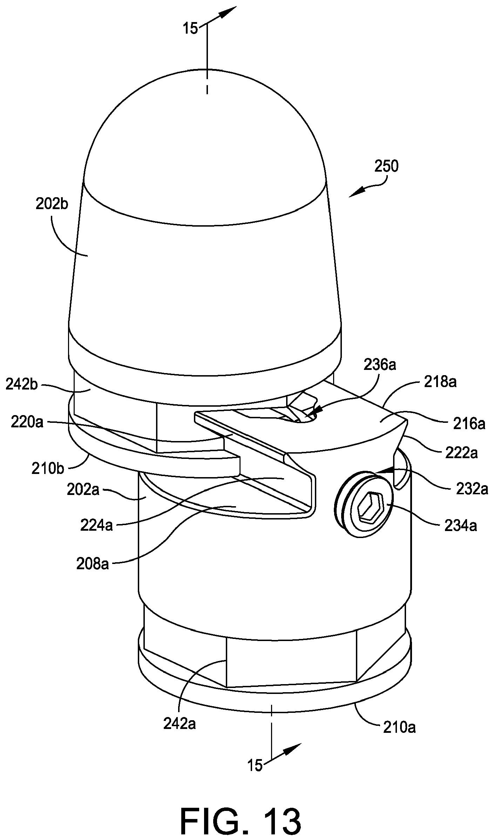

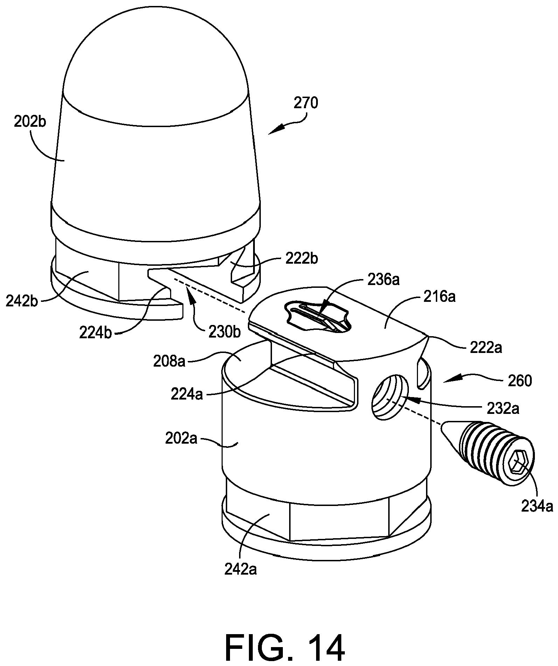

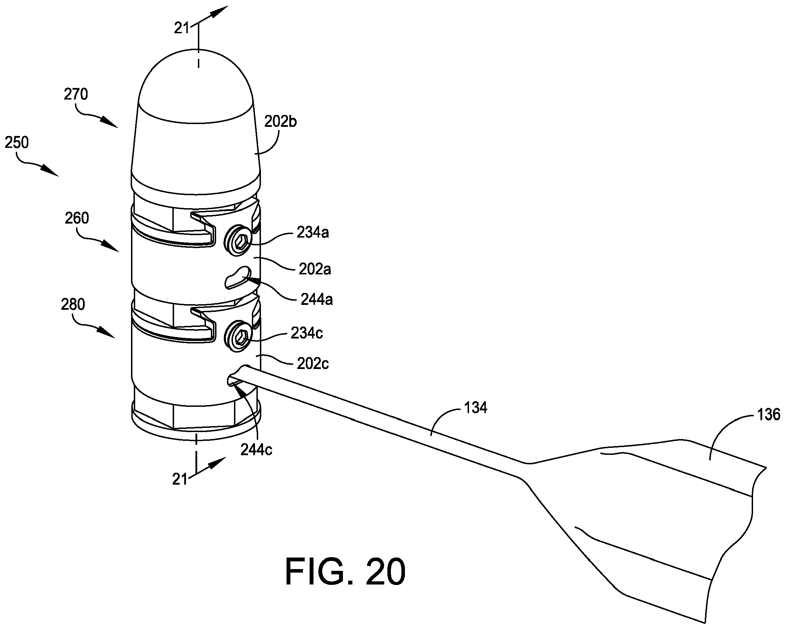

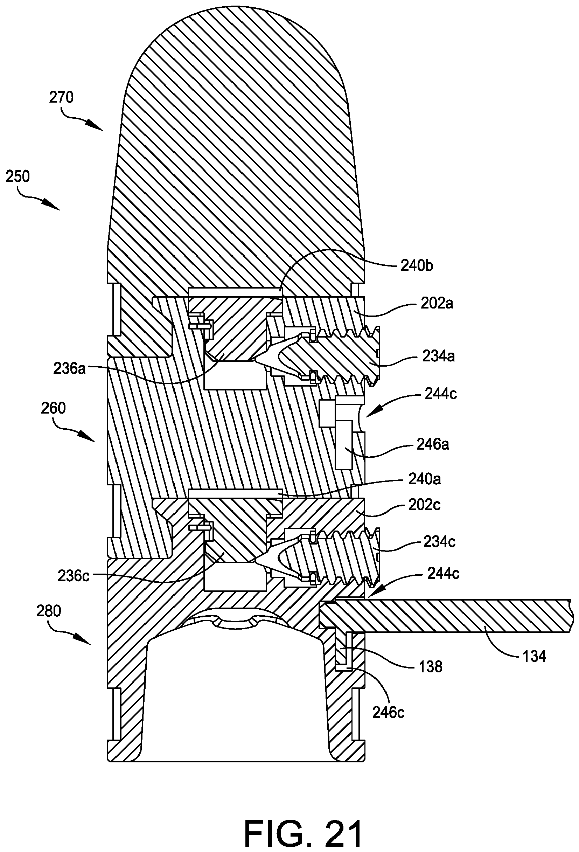

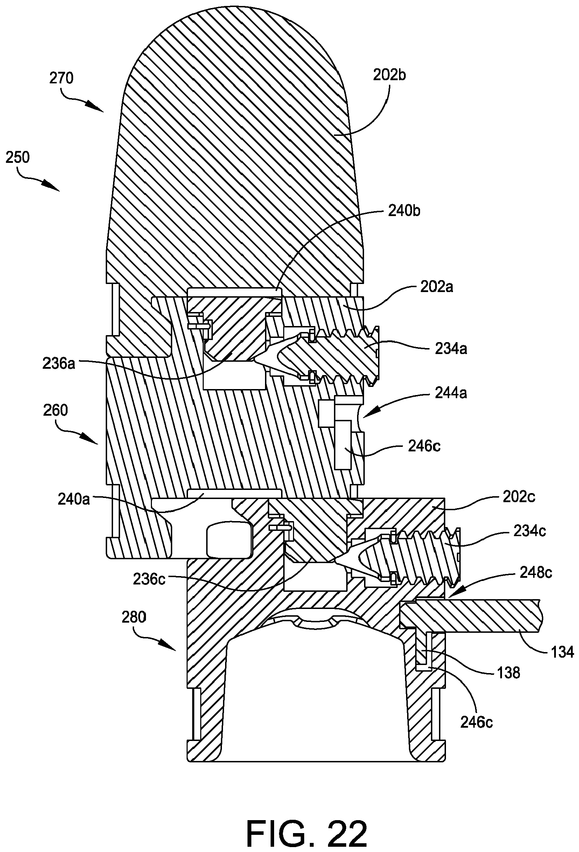

FIGS. 10-22 illustrate another example of a system 200 having a coupling mechanism in accordance with some embodiments. In some embodiments, the system 200 may be an ankle prosthesis including a stem 250, a tibia tray component 190, a tibial artificial joint surface 191, a talar component 192 with artificial surface, and a talar stem 193. The stem 250 may be a multi-component stem including two or more components that may be coupled by a coupling mechanism, which may be a dovetail coupling mechanism, as described herein. Although the components described herein are described with reference to an ankle prosthesis, the components, systems, and methods are not so limited, as one of ordinary skill in the art will understand that the components may be adapted for other prosthesis, including the hip, knee, and shoulder, to list only a few possible examples.

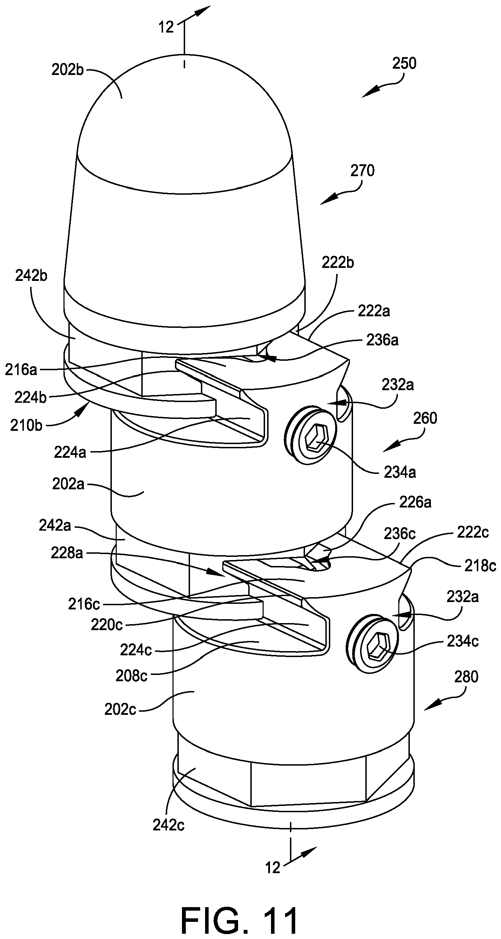

In the example illustrated in FIG. 11, the stem 250 includes a first component 270, which may be a top or leading component, a second component 260, which may be a middle component, and a third component 280, which may be a bottom component or another middle component. It should be understood that the second and third components may be identical or different depending on the length and configuration of the stem, as described herein. In some embodiments, the components 260, 270, 280 that may be coupled together either in situ or ex situ.

Each of the stem components 260, 270, 280 may include a body 202a, 202b, 202c having generally a cylindrical shape, with stem component 270 tapering along its length from a first diameter at leading end to a second diameter at its trailing end. In some embodiments, the second diameter is greater than the first diameter. It should be understood that stem components 260, 270, 280 may have other shapes or configurations (e.g., pyramidal, cubic, and/or oval, to list only a few examples). In addition, the components 260, 270, and/or 280 could be arched to reduce and/or prevent rotation, and could be of constant or varying cross-sectional widths. In some embodiments, the stem components 260, 270, 280 may include one or more anti-rotation features extending radially outward from the body 202a, 202b, 202c, as will be understood by one of ordinary skill in the art. Examples of such anti-rotation features are described in U.S. Patent Application Publication No. 2022/0280307 and U.S. Provisional Application No. 63/374,248, filed September 1, 2022, which are incorporated by reference herein in its entirety. The body of the components 260, 270, and/or 280 may be formed using an additive manufacturing process, such as Direct Metal Laser Sintering (DMLS) or Electron Beam Melting (EBM), to list only a few possibilities. Other conventional manufacturing processes, such as machining, molding, extruding, and combinations thereof, may also be used as will be understood by one of ordinary skill in the art.

In some embodiments, the body 202b of leading component 270 may further include a face 210b. The face 210b may define a pair of spaced apart rails 222b and 224b, disposed within channel 230b, configured to slide along spaced apart rails of another prosthesis component (e.g., stem component 160, 170 and/or another stem component of a multi-component stem) to form a dovetail coupling. The face 210b may further define a detent 240b adapted to engage a biasing member of another prosthesis component (e.g., stem component 160, 170 and/or another stem component of a multi-component stem) to lock the components together.

In some embodiments, the body 202a of stem component 260 may further include a first face 208a and a second face 210a. The first face 208a may, in some embodiments, define an engagement element 216a, such as a shelf or one or more flats, having a first edge 218a and a second edge 220a. The first 218a and second 220a edges define spaced apart rails 222a and 224a respectively, configured to slide along spaced apart rails within a channel disposed on the second face of another prosthesis component (e.g., prosthesis component 260, 280, and/or another stem component, such as leading component 270, of a multi-component stem) to form a dovetail coupling. The second face 210a may define a pair of spaced apart rails 226a and 228a, disposed within channel 230a, configured to slide along spaced apart rails of another prosthesis component (e.g., stem component 160, 170 and/or another stem component of a multi-component stem) to form a dovetail coupling. The second face 210a may further define a detent 240a adapted to engage a biasing member of another prosthesis component (e.g., stem component 260, 280 and/or another stem component of a multi-component stem) to lock the components together. One of ordinary skill in the art will understand that other engagement elements may be used instead of flats, rails, or shelfs.

In some embodiments, the body 202a of the prosthesis component 260 includes a securing mechanism 232a, wherein the securing mechanism is a hole extending inwardly from the outer surface perpendicular to longitudinal axis. The hole could be a blind hole or a through hole, and configured to receive a locking mechanism 234a at an angle perpendicular to the longitudinal axis of the body 202a. The securing mechanism 232a may comprise a threaded opening, partially threaded opening, and/or tapered opening, according to some embodiments. The locking mechanism 234a may include a fastener, such as a screw, clip, pin, bolt, nail, or plug just to give a few examples. The locking mechanism 234a may be threaded, partially threaded, and/or tapered to be compatible with the opening of the securing mechanism 232a as described above.

The body 202a may include a biasing member 236a on the first face 208a of the body 202a. According to some embodiments, the biasing member 236a may be configured to move along the longitudinal axis when in contact with the locking mechanism 234a. The biasing member is adapted to move into at least two positions to secure two components together. In further embodiments, the biasing member 236a, may include a clip 238a adapted to latch the first prosthesis component 260 with another prosthesis component (e.g., prosthesis component 260, 270, 280 and/or another stem component of a multi-component stem).

In some embodiments, the body 202c of stem component 270 may further include a first face 208c and a second face 210c. The first face 208c may, in some embodiments, define an engagement element 216c, such as a shelf or one or more flats, having a first edge 218c and a second edge 220c. The first 218c and second 220c edges define spaced apart rails 222c and 224c respectively, configured to slide along spaced apart rails within a channel disposed on the second face of another prosthesis component (e.g., prosthesis component 260, 280, and/or another stem component of a multi-component stem) to form a dovetail coupling. The second face 210c may define a pair of spaced apart rails 226c and 228c, disposed within channel 230c, configured to slide along spaced apart rails of another prosthesis component (e.g., stem component 160, 180 and/or another stem component of a multi-component stem) to form a dovetail coupling. The second face 210c may further define a detent 240c adapted to engage a biasing member of another prosthesis component (e.g., stem component 260, 280 and/or another stem component of a multi-component stem) to lock the components together. One of ordinary skill in the art will understand that other engagement elements may be used instead of flats, rails, or shelfs.

In some embodiments, the body 202c of the prosthesis component 280 includes a securing mechanism 232c, wherein the securing mechanism is a hole extending inwardly from the outer surface perpendicular to longitudinal axis. The hole could be a blind hole or a through hole, and configured to receive a locking mechanism 234c at an angle perpendicular to the longitudinal axis of the body 202c. The securing mechanism 232c may comprise a threaded opening, partially threaded opening, and/or tapered opening, according to some embodiments. The locking mechanism 234c may include a fastener, such as a screw, clip, pin, bolt, nail, or plug just to give a few examples. The locking mechanism 234c may be threaded, partially threaded, and/or tapered to be compatible with the opening of the securing mechanism 232c as described above.

The body 202c may include a biasing member 236c on the first face 208c of the body 202c. According to some embodiments, the biasing member 236c may be configured to move along the longitudinal axis when in contact with the locking mechanism 234c. The biasing member is adapted to move into at least two positions to secure two components together. In further embodiments, the biasing member 236c, may include a clip 238c adapted to latch the prosthesis component 280 with another prosthesis component (e.g., prosthesis component 260, 270, 280 and/or another stem component of a multi-component stem).

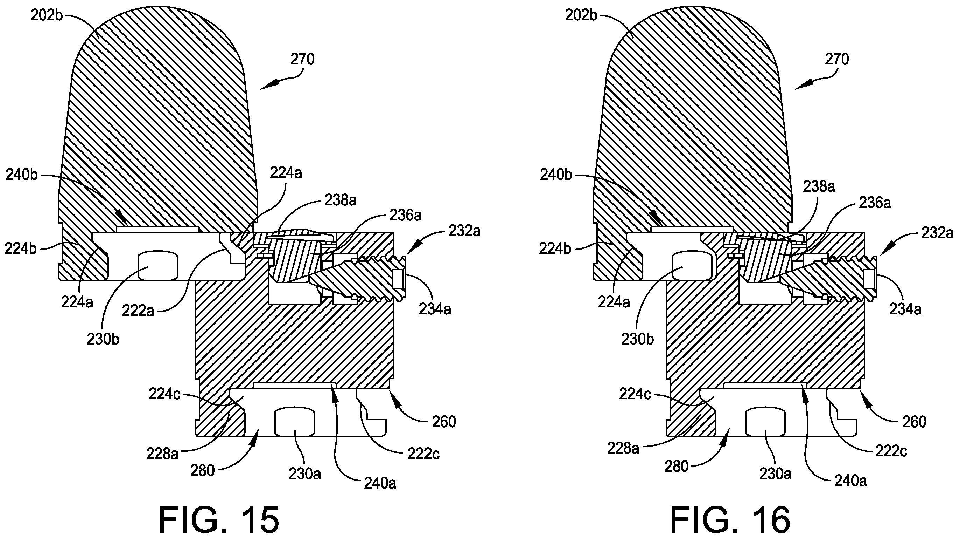

As best seen in FIGS. 15-18, a leading component 270, may be attached to components 260, 280. For example, the body 202b of component 270 may be inserted into a resected joint space between first and second bones, which may be a tibia and a talus. The component 270 may be advanced, at least partially, into an intramedullary canal form in one of the bones (e.g., the tibia). Component 260 may then be coupled to the component 270 by aligning spaced apart rails 222a, 224a and 222b, and 224b of the two components 260, 270. Once aligned component 260 may be pushed into engagement (e.g., by pushing in a first direction such as backward or in a posterior direction) so that engagement element 216a on the first face 208a of component 260 contacts the back end of the channel 230b. The locking mechanism 234a, such as a setscrew, may be installed in the securing mechanism 232a by hand and/or with an installation tool, according to some embodiments. When installed, the locking mechanism 232a pushes biasing member 236a into a detent 240b on the second face 210b of component 270 (or other compatible prosthesis component as discussed above). The clip 238a of the biasing member 236a may be used to lock the two components 260, 270 together once pushed into detent 240b. Stem component 260 may be installed by hand and/or by using a tool fitted around the body to install the two components 260, 270. One of ordinary skill in the art will understand that other engagement elements may be used, such as a spring-loaded detent or a depression and projections.

In some embodiments, the body 202a, 202b, 202c optionally includes a surface 242a, 242b configured to be engaged by a tool, such as a wrench, during the coupling of the first prosthesis component 260 to another component (e.g., to prosthesis component 270, 280 and/or to another stem component of a multi-component stem).

Bottom or middle component 280 may be coupled to middle component 260 by aligning spaced apart rails 226a, 228a and 222c, 224c for the two components 260 and 280. Once aligned, component 260 may be pushed into engagement (e.g., by pushing in a first direction such as backward) so that engagement element 216c contacts the back end of the channel 230a. Once component 280 is pushed into engagement, a locking mechanism 234c may be installed in the securing mechanism opening 232c. The installation of the locking mechanism 234c may push the biasing member 236c and clip 238c into the detent 240a locking the two components together and completing the coupling. In some embodiments, the body 202c includes a surface 242c configured to be engaged by a tool, such as a wrench, during the coupling of the prosthesis component 280 to another component (e.g., to first prosthesis component 260 and/or to another stem component of a multi-component stem).

In some embodiments, another component may be coupled to components 260, 270, or 280, such as a tibial tray component 190 for an ankle replacement system similar to the tibial platform 12 described in reference to FIG. 17 of U.S. Patent No. 8,715,362. Other components, such as one or of the tibia tray components 190 described in U.S. Patent Application No. 17/650,116, may also be used. In some embodiments, the tibial tray component 190 may define a channel for receiving an artificial articular surface 191, which may be configured to engage another articular surface, such as a talus or another artificial articular surface 192, as shown in FIG. 10. In some embodiments, one of the stem components 260, 270, and/or 280 may be coupled to the tray component 190 by aligning spaced apart rails of the stem component 260, 270, and/or 280 with an engagement element disposed on the tray component 190 similar to the dovetail coupling described above for the coupling of components 260 and 270, 280. In other embodiments, tray component 190 may be coupled to another stem component 260, 270, and/or 280 with a tapered connection. In the embodiment with a tapered connection for the tray component 190, a modified stem component, such as component 260, 270, or 280, would be provided without the channel and spaced apart rails as described above and would be adapted to fit with the tapered connection of the tray component 190.

Component 260 may be decoupled from component 270 by uninstalling the locking mechanism 234a and pulling the component 260 forward along the spaced apart rails 222a, 224a and 222b, 224b of both components. In some embodiments, a removal tool may be used to remove the locking mechanism 234a or force the component 260 out of contact with the back end of the channel 230b as will be understood by one of ordinary skill in the art. By uninstalling the locking mechanism 234a, the biasing member 236a and clip 238a are removed from the detent 240b unlocking the two components 260, 270 from each other and allowing the components to be slid apart on the spaced apart rails 222a, 224a and 222b, 224b.

The decoupling of components 260 and 280 may work in the same manner as the decoupling of components 260 and 270 described above. For example, component 260 may be decoupled from component 280 by uninstalling the locking mechanism 234c and pulling the component 280 forward (e.g., in an anterior direction) along the spaced apart rails 226a, 228a and 222c, 224c of both components. In some embodiments, a removal tool (not shown) may be used to remove the locking mechanism 234c or force the component 260 out of contact with the back end of the channel 230a as will be understood by one of ordinary skill in the art. By uninstalling the locking mechanism 234c, the biasing member 236c and clip 238c are removed from the detent 240a unlocking the two components 260 and 280 from each other and allowing the components to be slid apart on the spaced apart rails 226a, 228a and 222c, 224c.

In some embodiments, the decoupling and removal of the tibial tray component 190, from at least one other component (e.g., component 260, 270, and/ or 280) allows access to the internal engagement features of at least one other component 260, 270, 280. Removal of the tibial tray component 190, may be achieved with an impacting instrument(s) in a distal or caudal direction as will be understood by one of ordinary skill in the art. The internal engagement features of the components 260, 270, 280 may be spaced apart rails, such as the spaced apart rails 226a, 228a of component 260, and may be configured to engage a removal instrument (not shown). The removal instrument may engage the internal engagement features of components 260, 270, and/or 280, such as the spaced apart rails 226a, 228a of component 260, to facilitate removal. It is understood that the internal engagement features may also include holes, tabs, threads, and/or recesses to facilitate engagement with the removal instrument(s).

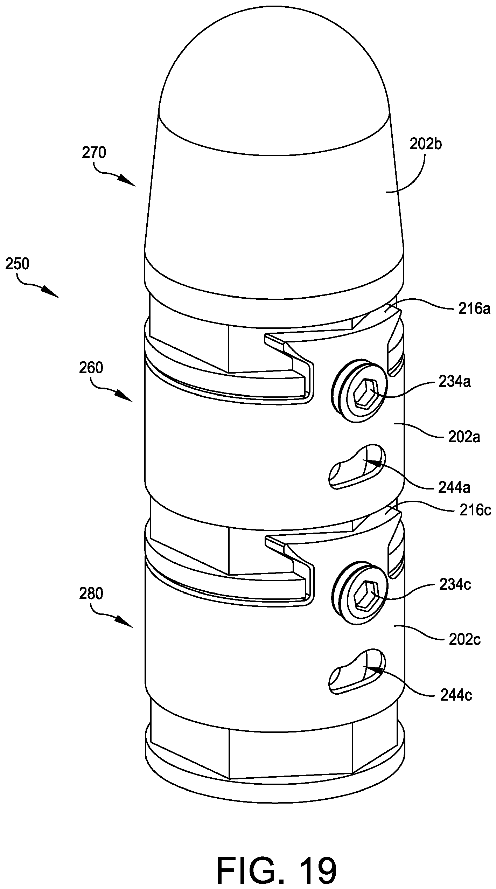

As best seen in FIGS. 19-22, stem components may include an extraction opening 244a, 244c in the body 202a, 202c configured to fit a separate tool, such as extraction tool 134, to allow for the decoupling and removal of at least one component, such as components 260, 270, and/or 280. In some embodiments, the extraction tool 134 may include a handle 136 disposed on a first end of the extraction tool 134 and an extraction mechanism 138 disposed on a second end of the extraction tool 134. The extraction mechanism 138, may in some embodiments, be a protrusion that extends at an angle from the shaft and adapted to fit inside of a recess 246a, 246c disposed inside of the extraction opening 244a 244c on body 202a, 202c. In some embodiments, the decoupling and removal of the stem components may include inserting the extraction tool 134 and extraction mechanism 138 into the extraction opening 244a, 244c and a recess 246a, 246c disposed on body 202a, 202c. Removal may then be facilitated by pulling away from (or in the posterior direction) the stem allowing the stem components to move along the spaced apart rails, such as spaced apart rails 226a, 228a and 222c, 224c of components 160 and 170 respectively.

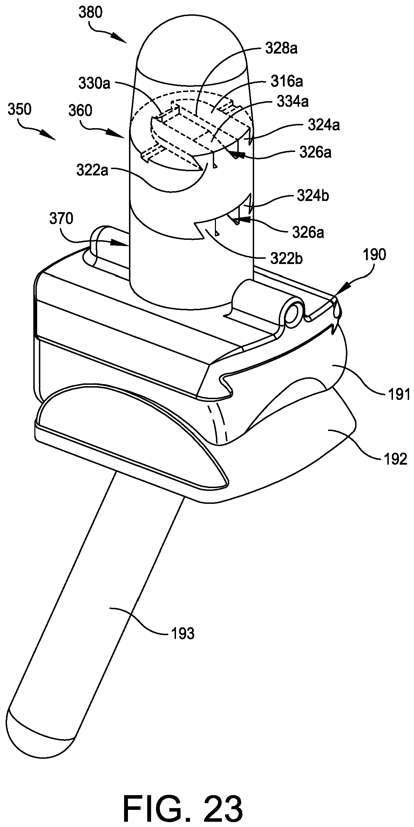

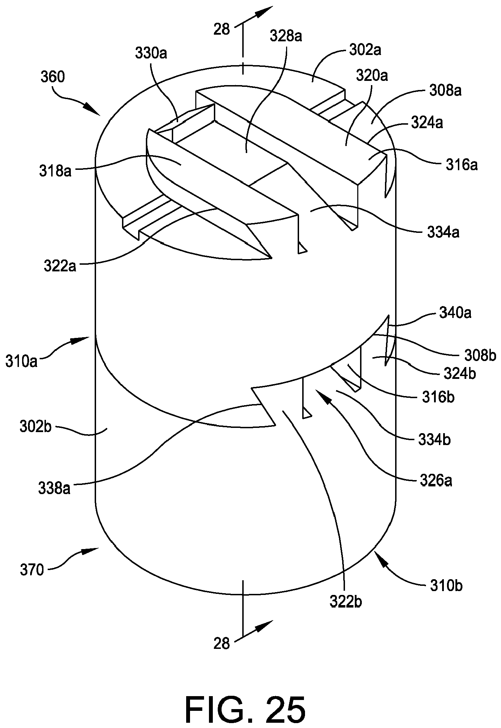

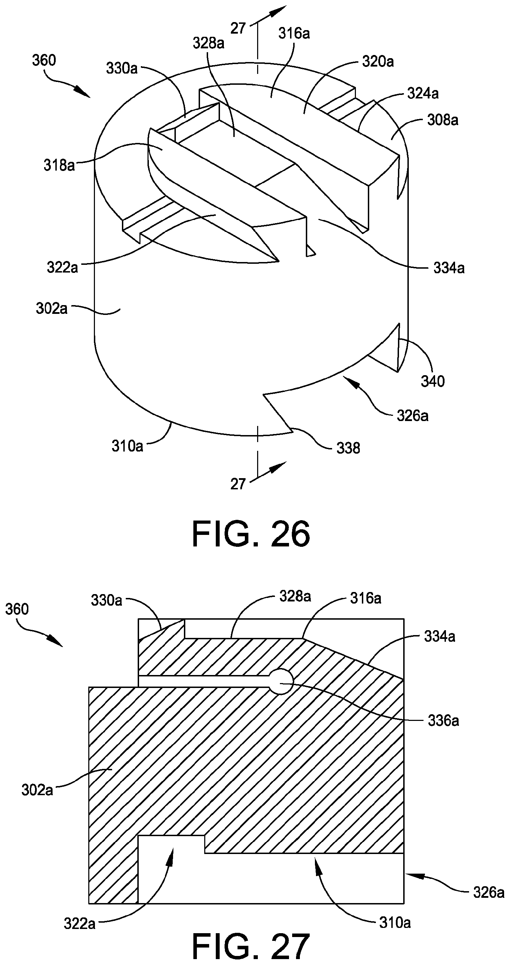

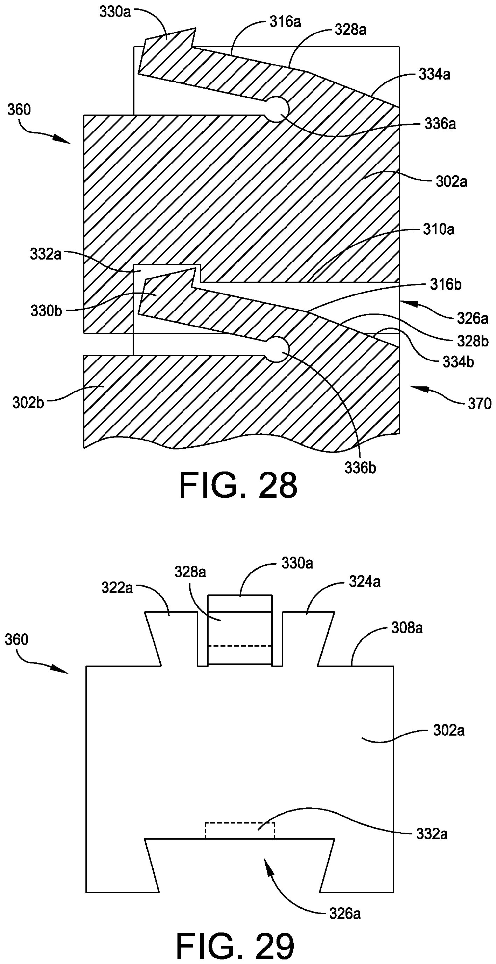

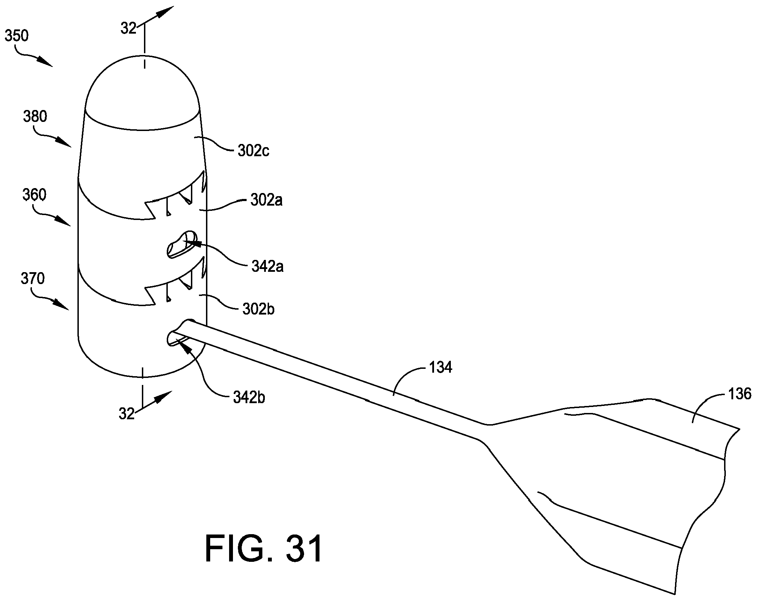

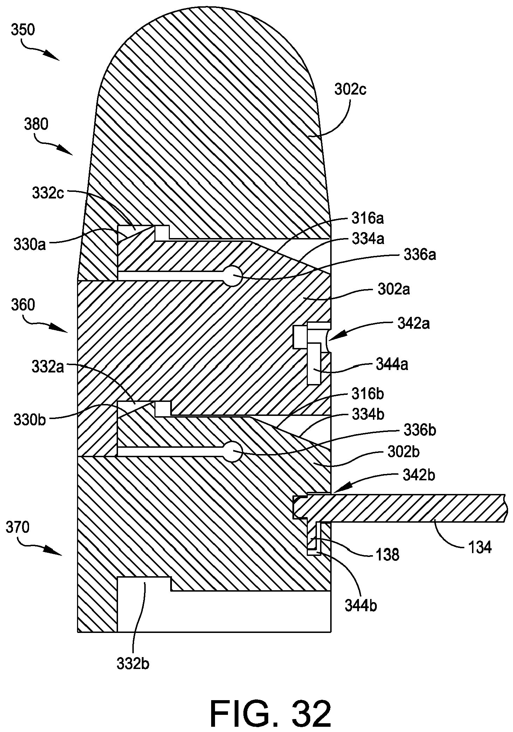

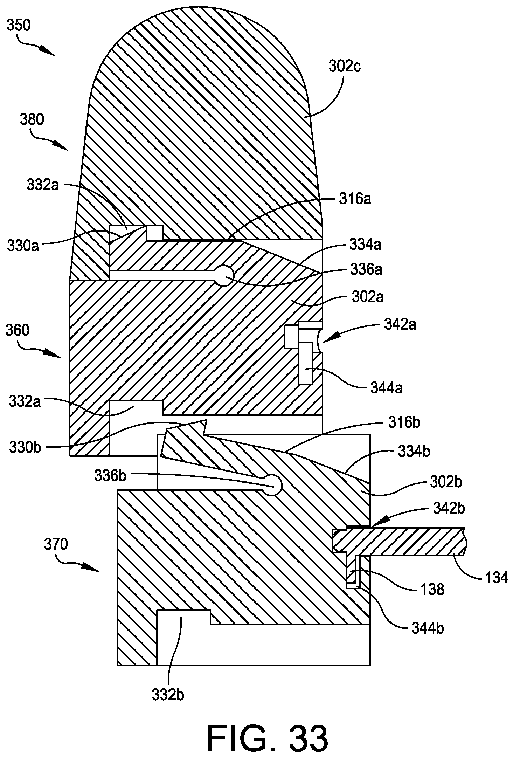

FIGS. 23-33 illustrate another example of a system 300 having a coupling mechanism in accordance with some embodiments. In some embodiments, the system 300 may be an ankle prosthesis including a stem 350, a tibia tray component 190, a tibial artificial joint surface 191, a talar component 192 with artificial surface, and a talar stem 193. The stem 350 may be a multi-component stem including two or more components that may be coupled by a coupling mechanism, which may be a dovetail coupling mechanism, as described herein. Although the components described herein are described with reference to an ankle prosthesis, the components, systems, and methods are not so limited, as one of ordinary skill in the art will understand that the components may be adapted for other prosthesis, including the hip, knee, and shoulder, to list only a few possible examples.

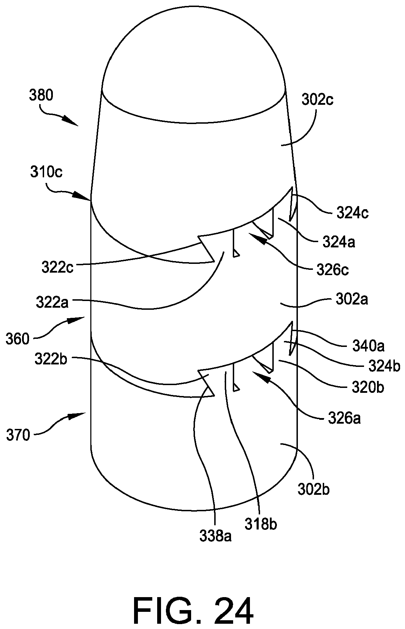

In the example illustrated in FIG. 24, the stem 350 includes a first component 380, which may be a top or leading component, a second component 360, which may be a middle component, and a third component 370, which may be a bottom or another middle component. It should be understood that the second and third components may be identical or different depending on the length and configuration of the stem, as described herein. In some embodiments, the components 360, 370, 380 may be coupled together either in situ or ex situ.

Each of the stem components 360, 370, 380 may include a body 302a, 302b, 302c having a generally cylindrical shape, with stem component 380 tapering along its length from a first diameter at leading end to a second diameter at its trailing end. In some embodiments, the second diameter is greater than the first diameter. It should be understood that stem component 360 may have other shapes or configurations (e.g., pyramidal, cubic, and/or oval, to list only a few examples). In addition, the stem components 360, 370, 380 could be arched to reduce and/or prevent rotation, and could be of constant or varying cross-sectional widths. In some embodiments, the stem components 360, 370, 380 may include one or more anti-rotation features extending radially outward from the body 302a, 302b, 302c, as will be understood by one of ordinary skill in the art. Examples of such anti-rotation features are described in U.S. Patent Application Publication No. 2022/0280307 and U.S. Provisional Application No. 63/374,248, filed September 1, 2022, which are incorporated by reference herein in its entirety. Body 302a, 302b, 302c may be formed using an additive manufacturing process, such as Direct Metal Laser Sintering (DMLS) or Electron Beam Melting (EBM), to list only a few possibilities. Other conventional manufacturing processes, such as machining, molding, extruding, and combinations thereof, may also be used as will be understood by one of ordinary skill in the art.

In some embodiments, body 320c of leading component 380 further include a face 310c. The face 310c may define a pair of spaced apart rails 322c and 324c, disposed within channel 326c, configured to slide along spaced apart rails of another prosthesis component (e.g., stem component 360, 370 and/or another stem component of a multi-component stem) to form a dovetail coupling.

In some embodiments, the body 302a of the prosthesis component 360 may further include a first face 308a and a second face 310a. The first face 308a may, in some embodiments, define an engagement element 316a having a first edge 318a and a second edge 320a. The first 318a and second 320a edges include spaced apart rails 322a and 324a configured to slide along spaced apart rails of another prosthesis component (e.g., prosthesis component 360, 370 and/or another stem component, such as leading component 380, of a multi-component stem) to form a dovetail coupling. One of ordinary skill in the art will understand that other engagement elements may be used instead of flats, rails, or shelfs.

In some embodiments, body 302a further includes a biasing mechanism 328a, which may include a tab 330a configured to fit inside of a detent on the second face of another prosthesis component (e.g., prosthesis component 360, 370 and/or another stem component, such as leading component 380, of a multi-component stem). The engagement element 316a may also include an angled slope 334a configured to allow a removal tool (not shown), such as a flat head screwdriver, access to release the tab 330a. In some embodiments, the tab 330a and body 302a define a cut out 336a allowing the tab to move in the longitudinal direction. This movement by the tab 330a provides the ability for the tab 330a to fit into a detent, such as detent 332b on component 370 or a detent of component 380, during one example of the coupling process or to decouple the two components with the removal tool. In some embodiments, the cut out 336a may be circular in shape, but it should be understood that the cut out 336a may have other shapes (e.g., pyramidal, cubic, rectangular and/or oval, to list only a few examples).

In some embodiments, the body 302b of the prosthesis component 370 may further include a first face 308b and a second face 310b. The first face 308b may, in some embodiments, define an engagement element 316b having a first edge 318b and a second edge 320b. The first 318b and second 320b edges include spaced apart rails 322b and 324b configured to slide along spaced apart rails of another prosthesis component (e.g., prosthesis component 360, 370 and/or another stem component, such as leading component 380, of a multi-component stem) to form a dovetail coupling. One of ordinary skill in the art will understand that other engagement elements may be used instead of flats, rails, or shelfs.

In some embodiments, body 302b further includes a biasing mechanism 328b, which may include a tab 330b configured to fit inside of a detent of another prosthesis component (e.g., prosthesis component 360, 370 and/or another stem component, such as leading component 380, of a multi-component stem). The engagement element 316b may also include an angled slope 334b configured to allow a removal tool (not shown), such as a flat head screwdriver, access to release the tab 330b. In some embodiments, the tab 330b and body 302b define a cut out 336b allowing the tab to move in the longitudinal direction. This movement by the tab 330b provides the ability for the tab 330b to fit into a detent, such as detent 332a on component 360, during one example of the coupling process or to decouple the two components with the removal tool. In some embodiments, the cut out 336b may be circular in shape, but it should be understood that the cut out 336b may have other shapes (e.g., pyramidal, cubic, rectangular and/or oval, to list only a few examples).

In some embodiments, a leading component 380, may be attached to components 360 and/or 370. For example, the body 302c of component 380 may be inserted into a resected joint space between first and second bones, which may be a tibia and a talus. The component 380 may be advanced, at least partially, into an intramedullary canal form in one of the bones (e.g., the tibia). Component 360 may then be coupled to the component 380 by aligning spaced apart rails 322a, 324a and 322c, and 324c of the two components 360, 380. Once aligned, component 360 may be pushed into engagement (e.g., by pushing in a first direction such as backward or in a posterior direction) so that engagement element 316a on the first face 308a of component 360 contacts the back end of the channel 326c. Once component 360 is pushed into engagement, the tab 330a of the biasing mechanism 328a snaps into the detent, similar to detent 332a, 332b, (not shown) completing the coupling of components 360, 380.

Bottom or middle component 370 may be coupled to component 360 by aligning the spaced apart rails 338a, 340a and 322b, 324b for the two components 360, 370. Once aligned, component 370 may be pushed into engagement (e.g., by pushing in a first direction such as backward or posterior direction) so that engagement element 316b contacts the back end of the channel 326a. Once component 370 is pushed into engagement, the tab 330b of the biasing mechanism 328b snaps into the detent 332a completing the coupling of components 360, 380.

In some embodiments, another component may be coupled to components 360, 370, or 380, such as a tibial tray component 190 for an ankle replacement system similar to the tibial platform 12 described in reference to FIG. 17 of U.S. Patent No. 8,715,362. Other components, such as one or of the tibia tray components 190 described in U.S. Patent Application No. 17/650,116, may also be used. In some embodiments, the tibial tray component 190 may define a channel for receiving an artificial articular surface 191, which may be configured to engage another articular surface, such as a talus or another artificial articular surface 192, as shown in FIG. 23. In some embodiments, one of the stem components 360, 370, and/or 380 may be coupled to the tray component 190 by aligning spaced apart rails of the stem component 360, 370, and/or 380 with an engagement element disposed on the tray component 190 similar to the dovetail coupling described above for the coupling of components 360 and 370, 380. In other embodiments, tray component 190 may be coupled to another stem component 360, 370, and/or 380 with a tapered connection. In the embodiment with a tapered connection for the tray component 190, a modified stem component, such as component 360, 370, or 380, would be provided without the channel and spaced apart rails as described above and would be adapted to fit with the tapered connection of the tray component 190

Component 370 may be decoupled from component 360 by inserting a removal tool (not shown) along the angled slope 334b and releasing the tab 330b out of the detent 332a. The cut out 336b provides the tab 330b room to move in the longitudinal axis and out of the detent 332a. Once the tab 330b is released the component 370 may be pulled forward along the spaced apart rails of both components 338a, 340a and 322b, 324b removing component 370 from component 360.

Component 380 may be decoupled from component 360 by inserting a removal tool (not shown) along the angled slope 334a and releasing the tab 330a out of the detent, similar to detent 332a, 332b, (not shown). The cut out 336a provides the tab 330a room to move in the longitudinal axis and out of the detent (not shown). Once the tab 330a is released the component 360 may be pulled forward along the spaced apart rails of both components 322a, 324a and 322c, 324c removing component 360 from component 380.

In some embodiments, the decoupling and removal of the tibial tray component 190, from at least one other component (e.g., component 360, 370, and/ or 380) allows access to the internal engagement features of at least one other component 360, 370, 380. Removal of the tibial tray component 190, may be achieved with an impacting instrument(s) in a distal or caudal direction as will be understood by one of ordinary skill in the art. The internal engagement features of the components 360, 370, 380 may be spaced apart rails, such as the spaced apart rails 338a, 340a of component 360, and may be configured and arranged to engage a removal instrument (not shown). The removal instrument may engage the internal engagement features of components 360, 370, and/or 380, such as the spaced apart rails 338a, 340a of component 360, to facilitate removal. It is understood that the internal engagement features may also include holes, tabs, threads, and/or recesses to facilitate engagement with the removal instrument(s).

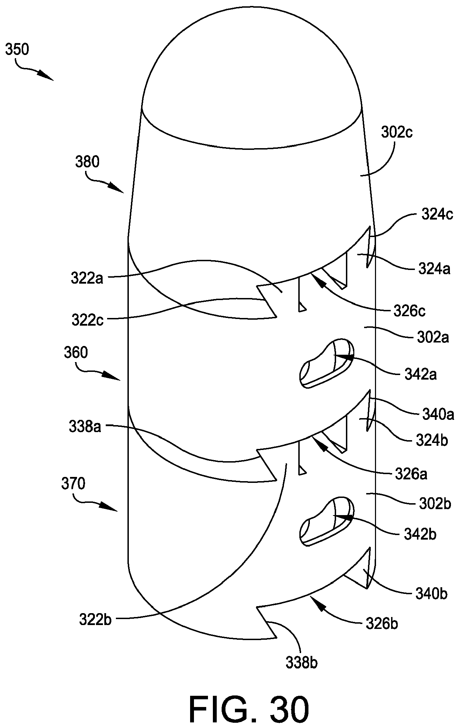

As best seen in FIGS. 30-33, stem components may include an extraction opening 342a, 342b in the body 302a, 302b configured to fit a separate tool, such as extraction tool 134, to allow for the decoupling and removal of at least one component, such as components 360, 370, and/or 380. In some embodiments, the extraction tool 134 may include a handle 136 disposed on a first end of the extraction tool 134 and an extraction mechanism 138 disposed on a second end of the extraction tool 134. The extraction mechanism 138, may in some embodiments, be a protrusion that extends at an angle from the shaft and adapted to fit inside of a recess 140a, 140b disposed inside of the extraction opening 342a 342b on body 302a, 302b. In some embodiments, the decoupling and removal of the stem components may include inserting the extraction tool 134 and extraction mechanism 138 into the extraction opening 342a, 342b and a recess 344a, 344b disposed on body 302a, 302b. Removal may then be facilitated by pulling away from (or in the posterior direction) the stem allowing the stem components to move along the spaced apart rails, such as spaced apart rails 338a, 340a and 322b, 324b of components 360 and 370 respectively.

In some embodiments, a system may include a first component, an engagement element, a biasing member, and a securing mechanism. The first component may have a first body extending from a first face to a second face. The engagement element may be disposed on the first face of the first body and may be configured to engage a second component. The biasing member may be disposed on the engagement element and may be configured to latch a second component to the first component. The securing mechanism may be configured to receive a locking mechanism at an angle with respect to a longitudinal axis. The locking mechanism may be adapted to interact with the biasing member.

In some embodiments, the first face on the first body may have a respective first edge and second edge.

In some embodiments, the securing mechanism may be configured to receive the locking mechanism at a perpendicular angle with respect to the longitudinal axis.

In some embodiments, the biasing member may be adapted to move in an up and a down direction.

In some embodiments, the engagement element may include a pair of spaced apart rails.

In some embodiments, the biasing member may include a clip.

In some embodiments, the locking mechanism may include a fastener configured to be received in an opening defined by the first body and adapted to interact with said biasing member. An interaction between the fastener and the biasing member may push the biasing member into a first position.

In some embodiments, the opening is at least partially threaded.

In some embodiments, the opening is at least partially tapered.

In some embodiments, the fastener may be a set screw configured to be received in the opening on the first body.

In some embodiments, the second component may include a second body extending from a first face to a second face. The second body may include rails on the first face of the second body. A detent may be disposed on a second face of the second body. The rails on the second body may be adapted to fit with the engagement element of the first component coupling the first and second components together.

In some embodiments, the biasing member, when pushed into the first positon by the fastener, may be adapted to lock the coupling of the first and second components together.

In some embodiments, the first component may include an angled slope on the first body sized to allow a removal tool access to the biasing member to decouple the first component from the second component.

In some embodiments, a system may include a tray configured to be coupled to the second component and an articular surface configured to be coupled to the tray.

In some embodiments, the first and second components may be formed using an additive manufacturing process.

In some embodiments, the bodies of the first and second components further comprise an extraction opening and a recess adapted to fit an extraction tool and extraction mechanism to facilitate the removal of the first and second components.

In some embodiments, a method may include coupling a first component to a second component by aligning a pair of rails on the first component to a pair of rails on the second component sliding the second component in a posterior direction to engage the pair of rails of the second component with the pair of rails on the first component.

In some embodiments, a method may include installing a locking mechanism into a securing mechanism disposed on an outer surface of the first component at an angle with respect to a longitudinal axis of the first component. The locking mechanism may be adapted to push a biasing member into a first position.

In some embodiments, the first and second components may be components of a stem.

In some embodiments, a method may include inserting the stem into an intramedullary canal formed in a tibia.

In some embodiments, a method may include coupling a tibial tray component to the stem.

In some embodiments, a method may include coupling the second component to another component.

In some embodiments, a respective body of each of the first and second components may include a detent on a respective second face.

In some embodiments, the biasing member may include a fastener configured to lock the first and second components together.

In some embodiments, a method may include decoupling a first component from a second component. The first and second components may both include a respective body. The respective bodies may include respective spaced apart rails of the first and second component. The decoupling may include unlocking the first and second components by uninstalling a locking mechanism from a securing mechanism disposed, at an angle with respect to a longitudinal axis of the first component on an outer surface of the first component. The removal of the locking mechanism may move a biasing member into a second position. The decoupling may include moving the first and second components apart by sliding the respective spaced apart rails away from each other by pulling an extraction tool handle in a posterior direction.

In some embodiments, a system may include a first component, a pair of rails, and a biasing member. The first component may include a first body extending from a first face to a second face. The pair of rails may be disposed on the first face of the first body. The pair of rails may be configured to engage a second component. The biasing member may be disposed on the first face of the first body and may be configured to latch the first and second component together.

In some embodiments, the first body may include an angled slope on the first face of the first body and may include a detent on the second face of the first body configured to receive a tab.

In some embodiments, the biasing member may include a tab and a cut out to allow movement of the tab.

In some embodiments, the cut out may be circular in shape.

In some embodiments, the second component may include a second body and a pair of rails. The second body may extend from a first face to a second face. The pair of rails disposed on the first face of the second body. The rails may be configured to slide along the rails of the first component coupling the first and second components together.

In some embodiments, the second body may include an angled slope, disposed on the first face of the second body, and may include a detent on the second face of the second body, configured to receive a tab.

In some embodiments, the second component may include a second biasing member disposed on the first face of the second component. The second biasing member may include a second tab on a top side of the second biasing member adapted to lock the second component and may include at least one other component together.

In some embodiments, the second biasing member may include a second cut out on a bottom side of the second biasing member allowing movement of the second biasing member.

In some embodiments, a system may include a removal tool configured to release the second tab decoupling the first and second components.

In some embodiments, a system may include a tray configured to be coupled to the second component and may include an articular surface configured to be coupled to the tray.

In some embodiments, the first and second components may be formed using an additive manufacturing process.

In some embodiments, a method may include coupling a first component and a second component together by aligning a respective pair of rails of the first and second components and locking the first and second components together with an engagement element disposed on a first face of the second component. The engagement element may include a tab, a cut out, an angled slope, and a ledge. The tab may be configured to fit inside of a detent. The cut out on the bottom side of the tab may be configured to allow the tab to bend. The angled slope may be adapted to allow a removal tool access. The ledge may be on the first end of the tab. The ledge may be adapted to lock inside of a detent on a bottom end of the first component.

In some embodiments, a method may include coupling the second component to another component.

In some embodiments, the first and second components may be components of a stem.

In some embodiments, a method may include inserting the stem into an intramedullary canal formed in a tibia.

In some embodiments, a method may include coupling a tibial tray component to the stem.

In some embodiments, a method may include decoupling a first component from a second component. The first and second components may include a respective body. The respective bodies may include respective spaced apart rails. The decoupling may include sliding a removal tool along an angled slope on the body of the second component. The removal tool may be adapted to engage a tab on the second component and may remove the tab from a detent on a second face of the first component. If the tab is removed from the detent, moving the respective spaced apart rails of the first and second component apart by pulling an extraction tool handle in a posterior direction completing the decoupling.

Although the components, systems, kits, and methods have been described in terms of exemplary embodiments, they are not limited thereto. Rather, the appended claims should be construed broadly, to include other variants and embodiments of the components, systems, kits, and methods, which may be made by those skilled in the art without departing from the scope and range of equivalents.

Further disclosed herein is the subject-matter of the following clauses:
1. A system, comprising:
   a first component having:
   a first body extending from a first face to a second face;
   an engagement element disposed on the first face of the first body and configured to engage a second component;
   a biasing member disposed on the engagement element and configured to latch a second component to the first component; and
   a securing mechanism configured to receive a locking mechanism at an angle with respect to a longitudinal axis, wherein the locking mechanism is adapted to interact with the biasing member.
2. The system of clause 1, wherein the first face on the first body has a respective first edge and second edge.
3. The system of clause 1 or of any of the preceding clauses, wherein the securing mechanism is configured to receive the locking mechanism at a perpendicular angle with respect to the longitudinal axis.
4. The system of clause 1 or of any of the preceding clauses, wherein the biasing member is adapted to move in an up and a down direction.
5. The system of clause 1 or of any of the preceding clauses, wherein the engagement element includes a pair of spaced apart rails.
6. The system of clause 1 or of any of the preceding clauses, wherein the biasing member includes a clip.
7. The system of clause 1 or of any of the preceding clauses, wherein the locking mechanism includes a fastener configured to be received in an opening defined by the first body and adapted to interact with said biasing member, wherein an interaction between the fastener and the biasing member pushes the biasing member into a first position.
8. The system of clause 7, wherein the opening is at least partially threaded.
9. The system of clause 7 or of any of clauses 7-8, wherein the opening is at least partially tapered.
10. The system of clause 7 or of any of clauses 7-9, wherein the fastener is a set screw configured to be received in the opening on the first body.
11. The system of clause 7 or of any of clauses 7-10, wherein the second component further includes a second body extending from a first face to a second face, the second body including rails on the first face of the second body, and a detent disposed on a second face of the second body, wherein the rails on the second body are adapted fit with the engagement element of the first component coupling the first and second components together.
12. The system of clause 11, wherein the biasing member, when pushed into the first positon by the fastener, is adapted to lock the coupling of the first and second components together.
13. The system of clause 12, wherein the first component further comprises an angled slope on the first body sized to allow a removal tool access to the biasing member to decouple the first component from the second component.
14. The system of clause 11 or of any of clauses 11-13, further comprising:
   a tray configured to be coupled to the second component; and
   an articular surface configured to be coupled to the tray.
15. The system of clause 11 or of any of clauses 11-13, wherein the first and second components are formed using an additive manufacturing process.
16. The system of clause of clause 13, wherein the bodies of the first and second components further comprise an extraction opening and a recess adapted to fit an extraction tool and extraction mechanism to facilitate the removal of the first and second components.
17. A method, comprising:
   coupling a first component to a second component by:
   aligning a pair of rails on the first component to a pair of rails on the second component; and
   sliding the second component in a posterior direction to engage the pair of rails of the second component with the pair of rails on the first component.
18. The method of clause 16, further comprising installing a locking mechanism into a securing mechanism disposed on an outer surface of the first component at an angle with respect to a longitudinal axis of the first component, wherein the locking mechanism is adapted to push a biasing member into a first position.
19. The method of clause 17, wherein the first and second components are components of a stem.
20. The method of clause 19, further comprising inserting the stem into an intramedullary canal formed in a tibia.
21. The method of clause 20, further comprising coupling a tibial tray component to the stem.
22. The method of clause 17, further comprising coupling the second component to another component.
23. The method of clause 17, wherein a respective body of each of the first and second components includes a detent on a respective second face.
24. The method of clause 17, wherein the biasing member comprises a fastener configured to lock the first and second components together.
25. A method comprising:
   decoupling a first component from a second component, wherein the first and second components both include a respective body, the respective bodies further including respective spaced apart rails of the first and second component, the decoupling comprising:
      unlocking the first and second components by uninstalling a locking mechanism
   from a securing mechanism disposed, at an angle with respect to a longitudinal axis of the first component on an outer surface of the first component, wherein the removal of the
   locking mechanism moves a biasing member into a second position; and
      moving the first and second components apart by sliding the respective spaced apart rails away from each other by pulling an extraction tool handle in a posterior direction.
26. A system, comprising:
   a first component having:
   a first body extending from a first face to a second face;
   a pair of rails disposed on the first face of the first body, wherein the pair of rails are configured to engage a second component; and
   a biasing member disposed the first face of the first body and configured to latch the first and second component together.
27. The system of clause 26, wherein the first body further comprises an angled slope on the first face of the first body and a detent on the second face of the first body configured to receive a tab.
28. The system of clause 27, wherein the biasing member includes a tab and a cut out to allow movement of the tab.
29. The system of clause 28, wherein the cut out is circular in shape.
30. The system of clause 26 or of any of clauses 26-29, wherein the second component includes:
   a second body extending from a first face to a second face;
   a pair of rails disposed on the first face of the second body, wherein the rails are configured to slide along the rails of the first component, coupling the first and second components together.
31. The system of clause 30, wherein the second body further comprises an angled slope, disposed on the first face of the second body, and a detent on the second face of the second body, configured to receive a tab.
32. The system of clause 30, wherein the second component comprises a second biasing member disposed on the first face of the second component, wherein the second biasing member includes a second tab on a top side of the second biasing member adapted to lock the second component and at least one other component together.
33. The system of clause 32, wherein the second biasing member further includes a second cut out on a bottom side of the second biasing member allowing movement of the second biasing member.
34. The system of clause 33, further including a removal tool configured to release the second tab decoupling the first and second components.
35. The system of clause 26 or of any of clauses 26-34, further comprising:
   a tray configured to be coupled to the second component; and
   an articular surface configured to be coupled to the tray.
36. The system of clause 26 or of any of clauses 26-35, wherein the first and second components are formed using an additive manufacturing process.
37. The system of clause of clause 33, wherein the bodies of the first and second components further comprise an extraction opening and a recess adapted to fit an extraction tool and extraction mechanism to facilitate the removal of the first and second components.
38. A method, comprising:
   coupling a first component and a second component together by:
   aligning a respective pair of rails of the first and second components;
   locking the first and second components together with an engagement element disposed on a first face of the second component, the engagement element comprising:
      a tab configured to fit inside of a detent;
      a cut out on the bottom side of the tab configured to allow the tab to bend;
      an angled slope adapted to allow a removal tool access; and
      a ledge on the first end of the tab, wherein the ledge is adapted to lock inside of a detent on a bottom end of the first component.
39. The method of clause 38, further comprising coupling the second component to another component.
40. The method of clause 38, wherein the first and second components are components of a stem.
41. The method of clause 40, further comprising inserting the stem into an intramedullary canal formed in a tibia.
42. The method of clause 41, further comprising coupling a tibial tray component to the stem.
43. A method, comprising:
   decoupling a first component from a second component, wherein the first and second components include a respective body, the respective bodies further including respective spaced apart rails, the decoupling comprising:
   sliding a removal tool along an angled slope on the body of the second component, wherein the removal tool is adapted to engage a tab on the second component and remove the tab from a detent on a second face of the first component; and
   once the tab is removed from the detent, moving the respective spaced apart rails of the first and second component apart by pulling an extraction tool handle in a posterior direction completing the decoupling.

## Claims

1. A system, comprising:
a first component having:
a first body extending from a first face to a second face;
an engagement element disposed on the first face of the first body and configured to engage a second component;
a biasing member disposed on the engagement element and configured to latch a second component to the first component; and
a securing mechanism configured to receive a locking mechanism at an angle with respect to a longitudinal axis, wherein the locking mechanism is adapted to interact with the biasing member.

2. The system of claim 1, wherein the first face on the first body has a respective first edge and second edge, and/or wherein the securing mechanism is configured to receive the locking mechanism at a perpendicular angle with respect to the longitudinal axis, and/or wherein the biasing member is adapted to move in an up and a down direction, and/or wherein the engagement element includes a pair of spaced apart rails, and/or wherein the biasing member includes a clip.

3. The system of any of the preceding claims, wherein the locking mechanism includes a fastener configured to be received in an opening defined by the first body and adapted to interact with said biasing member, wherein an interaction between the fastener and the biasing member pushes the biasing member into a first position.

4. The system of claim 3, wherein the opening is at least partially threaded, and/or wherein the opening is at least partially tapered, and/or wherein the fastener is a set screw configured to be received in the opening on the first body.

5. The system of claim 3, wherein the second component further includes a second body extending from a first face to a second face, the second body including rails on the first face of the second body, and a detent disposed on a second face of the second body, wherein the rails on the second body are adapted fit with the engagement element of the first component coupling the first and second components together.

6. The system of claim 5, wherein the biasing member, when pushed into the first positon by the fastener, is adapted to lock the coupling of the first and second components together.

7. The system of claim 6, wherein the first component further comprises an angled slope on the first body sized to allow a removal tool access to the biasing member to decouple the first component from the second component, wherein the bodies of the first and second components optionally further comprise an extraction opening and a recess adapted to fit an extraction tool and extraction mechanism to facilitate the removal of the first and second components.

8. The system of any of claims 5-7, further comprising:
a tray configured to be coupled to the second component; and
an articular surface configured to be coupled to the tray; and/or wherein the first and second components are formed using an additive manufacturing process.

9. A system, comprising:
a first component having:
a first body extending from a first face to a second face;
a pair of rails disposed on the first face of the first body, wherein the pair of rails are configured to engage a second component; and
a biasing member disposed the first face of the first body and configured to latch the first and second component together.

10. The system of claim 9, wherein the first body further comprises an angled slope on the first face of the first body and a detent on the second face of the first body configured to receive a tab, wherein the biasing member includes a tab and a cut out to allow movement of the tab, wherein the cut out optionally is circular in shape.

11. The system of any of claims 9-10, wherein the second component includes:
a second body extending from a first face to a second face;
a pair of rails disposed on the first face of the second body, wherein the rails are configured to slide along the rails of the first component, coupling the first and second components together.

12. The system of claim 11, wherein the second body further comprises an angled slope, disposed on the first face of the second body, and a detent on the second face of the second body, configured to receive a tab.

13. The system of claim 11, wherein the second component comprises a second biasing member disposed on the first face of the second component, wherein the second biasing member includes a second tab on a top side of the second biasing member adapted to lock the second component and at least one other component together, wherein optionally the second biasing member further includes a second cut out on a bottom side of the second biasing member allowing movement of the second biasing member.

14. The system of any of claims 9-13, further comprising:
a tray configured to be coupled to the second component; and
an articular surface configured to be coupled to the tray.

15. The system of any of claims 9-14, wherein the first and second components are formed using an additive manufacturing process.

16. The system of claim 13, further including a removal tool configured to release the second tab decoupling the first and second components, and/or wherein the bodies of the first and second components further comprise an extraction opening and a recess adapted to fit an extraction tool and extraction mechanism to facilitate the removal of the first and second components.
